# EUROPEAN PATENT APPLICATION

(11) **EP 4 588 425 A1**
(43) Date of publication of application: **23.07.2025**
(21) Application number: 23865299.4
(22) Date of filing: 31.08.2023
(51) Int. Cl.: A61B 3/10

(54) **LACRIMAL LAYER EVALUATION DEVICE, LACRIMAL LAYER EVALUATION PROGRAM, AND LACRIMAL LAYER EVALUATION METHOD**

(30) Priority: 14.09.2022 JP 2022146234
(71) Applicant: KOWA COMPANY, LTD., Naka-ku Nagoya-shi Aichi 460-8625 (JP)
(72) Inventor: TANAKA Shin, Chofu-shi Tokyo 182-0021 (JP)
(74) Representative: Mathys & Squire
(86) International application number: PCT/JP2023/031717
(87) International publication number: WO 2024/057943

(57) **Abstract**

To improve determination accuracy by subdividing a captured image and to evaluate a lacrimal fluid layer while considering a temporal change in a lacrimal fluid layer, captured video data obtained by capturing the image of the eye to be examined is acquired, a plurality of determination target frames are extracted from the captured video data based on a predetermined extraction rule, a plurality of pieces of sector image data having a predetermined size are extracted from the determination target frames, whether each piece of sector image data is an image indicating a predetermined lacrimal fluid state is determined, the determination target frame is determined as a candidate image indicating the predetermined lacrimal fluid state when the number of pieces of sector image data determined as an image indicating the predetermined lacrimal fluid state exceeds a predetermined threshold value, and the eye to be examined shown in the captured video data is determined as the predetermined lacrimal fluid state when the determination target frames are arranged in time series and the determination target frames determined as a candidate image indicating the predetermined lacrimal fluid state satisfy a predetermined continuous condition.

## Description

### Technical Field

The present invention relates to a lacrimal fluid layer evaluation device, a lacrimal fluid layer evaluation program, and a lacrimal fluid layer evaluation method for analyzing a condition of an eye to be examined.

### Background Art

In the related arts, various methods are proposed in which an image of an eye to be examined is captured and a state of the eye to be examined is evaluated by a computer using the captured image. For example, Patent Literature 1 is proposed as an ophthalmic device for diagnosing dry eye.

Patent Literature 1 discloses an ophthalmic device that captures a color image of an interference pattern formed in a lacrimal fluid layer of an eye to be examined with illumination light, decomposes the captured color image into a plurality of color components, determines an interference fringe appearing in each color component based on a signal of each decomposed color component, and calculates a value indicating a progress state of dry eye based on the number of interference fringes determined for each color component or a signal level change of the determined interference fringe.

### Citation List

### Patent Literature

Patent Literature 1: JP 2005-211173 A

### Summary of Invention

### Technical Problem

Meanwhile, various techniques have been proposed for determining whether the eye to be examined is in a predetermined lacrimal fluid state such as dry eye from a captured image obtained by capturing an image of the eye to be examined as in the ophthalmic device described in Patent Literature 1 described above, and in general, a technique for evaluating one entire ophthalmic image has an advantage that analysis time is short because a result can be obtained by analyzing only one image but has disadvantages such as (1) diagnosis support capability is low as presence of an extraction target can be predicted but a location of a part of the image the extraction target cannot be identified, (2) it is extremely difficult to extract a structure or a characteristic of a target located in only a small part of the image, and (3) a noise element (a region of eyelashes with strong contrast or a contour of a pupil of an interference fringe image) can be erroneously considered as an extraction target.

Even if a predetermined lacrimal fluid state such as dry eye can be evaluated in one ophthalmic image, there is a problem that determination of whether the patient has dry eye from the ophthalmic image. There is also a problem that it is not reliable to evaluate a predetermined lacrimal fluid state with one ophthalmic image considering a case where a lacrimal fluid state is erroneously detected from only one ophthalmic image by chance or a case where a condition for diagnosis is that a predetermined lacrimal fluid state lasts for a certain period of time.

The present invention has been made in view of the above problems, and an object of the present invention is to provide a lacrimal fluid layer evaluation device, a lacrimal fluid layer evaluation program, and a lacrimal fluid layer evaluation method in which a captured image is subdivided as to improve determination accuracy and consider temporal changes of a lacrimal fluid layer.

### Solution to Problem

A lacrimal fluid layer evaluation device according to the present invention is a lacrimal fluid layer evaluation device for evaluating a lacrimal fluid state of an eye to be examined based on captured video data obtained by capturing an image of the eye to be examined, the device including: a captured video data acquisition unit that acquires the captured video data obtained by capturing the image of the eye to be examined; a determination target frame extraction unit that extracts a plurality of determination target frames from the captured video data based on a predetermined extraction rule; a frame unit determination unit that determines whether the determination target frame is a candidate image indicating a predetermined lacrimal fluid state based on a predetermined determination condition; and a video unit determination unit that determines that the eye to be examined shown in the captured video data is in the predetermined lacrimal fluid state when the determination target frames are arranged in time series and the determination target frames determined as a candidate image indicating the predetermined lacrimal fluid state satisfy a predetermined continuous condition.

In the lacrimal fluid layer evaluation device according to the present invention, further, the frame unit determination unit sets and stores a threshold value relating to the number of pieces of sector image data for determining that the determination target frame is an image indicating the predetermined lacrimal fluid state for each type of a lacrimal fluid state as a determination target.

In the lacrimal fluid layer evaluation device according to the present invention, further, the sector unit determination unit extracts the sector image data so that at least a part of an image region is overlapped on another piece of sector image data for each of the plurality of pieces of sector image data.

The lacrimal fluid layer evaluation device according to the present invention, further including: a display processing unit that generates a display screen to be displayed on the display device, in which the display processing unit displays a display indicating that the determination target frame is an image indicating the predetermined lacrimal fluid state to be overlapped on the determination target frame so that a corresponding region of the determination target frame as an extraction source of the sector image data determined as an image indicating the predetermined lacrimal fluid state by the sector unit determination unit can be recognized.

The lacrimal fluid layer evaluation device according to the present invention, further including: a display processing unit that generates a display screen to be displayed on the display device, in which, when the determination target frames are arranged in time series, the display processing unit displays a graph of temporal change in the number of pieces of sector image data determined as an image indicating the predetermined lacrimal fluid state.

In the lacrimal fluid layer evaluation device according to the present invention, further, when the determination target frames are divided into a plurality of aggregation regions and then arranged in time series, the display processing unit aggregates the number of pieces of sector image data determined as an image indicating the predetermined lacrimal fluid state for each of the aggregation regions and displays a graph of temporal change for each of the aggregation regions.

In the lacrimal fluid layer evaluation device according to the present invention, further, when the determination target frames are arranged in time series, the video unit determination unit determines, as the continuous condition, a case where the determination target frames determined as a candidate image indicating the predetermined lacrimal fluid state continuously appear for a predetermined number or more set in advance.

In the lacrimal fluid layer evaluation device according to the present invention, further, when the determination target frames are arranged in time series and extracted by the number of frames set in advance at a freely selected point, the video unit determination unit determines, as the continuous condition, a case where the determination target frames determined as a candidate image indicating the predetermined lacrimal fluid state appear by a predetermined ratio or more in an extracted range.

In the lacrimal fluid layer evaluation device according to the present invention, further, when the determination target frames are arranged in time series, the video unit determination unit determines, as the continuous condition, a case where the determination target frames determined as a candidate image continuously appear in a period from when the determination target frame determined as a candidate image indicating the predetermined lacrimal fluid state appears until an eyelid is closed.

A lacrimal fluid layer evaluation program according to the present invention is a lacrimal fluid layer evaluation program for causing a computer to realize processing of evaluating a lacrimal fluid state of an eye to be examined based on captured video data obtained by capturing an image of the eye to be examined, the program causing the computer to realize: a captured video data acquisition function of acquiring the captured video data obtained by capturing the image of the eye to be examined; a determination target frame extraction function of extracting a plurality of determination target frames from the captured video data based on a predetermined extraction rule; a frame unit determination function of determining whether the determination target frame is a candidate image indicating a predetermined lacrimal fluid state based on a predetermined determination condition; and a video unit determination function of determining that the eye to be examined shown in the captured video data is in the predetermined lacrimal fluid state when the determination target frames are arranged in time series and the determination target frames determined as a candidate image indicating the predetermined lacrimal fluid state satisfy a predetermined continuous condition.

A lacrimal fluid layer evaluation method according to the present invention is a lacrimal fluid layer evaluation method for evaluating a lacrimal fluid state of an eye to be examined based on captured video data obtained by capturing an image of the eye to be examined by a computer, the method including: a captured video data acquisition step of acquiring the captured video data obtained by capturing the image of the eye to be examined; a determination target frame extraction step of extracting a plurality of determination target frames from the captured video data based on a predetermined extraction rule; a frame unit determination step of determining whether the determination target frame is a candidate image indicating a predetermined lacrimal fluid state based on a predetermined determination condition; and a video unit determination step of determining that the eye to be examined shown in the captured video data is in the predetermined lacrimal fluid state when the determination target frames are arranged in time series and the determination target frames determined as a candidate image indicating the predetermined lacrimal fluid state satisfy a predetermined continuous condition.

### Advantageous Effects of Invention

One or more deficiencies are solved by the embodiments of the present application.

### Brief Description of Drawings

Fig. 1 is a block diagram illustrating an example of a configuration of a lacrimal fluid layer evaluation device and peripheral devices thereof according to at least one of embodiments of the present invention.
Fig. 2 is an explanatory diagram illustrating an example of a configuration of a capturing device according to at least one of the embodiments of the present invention.
Fig. 3 is a block diagram illustrating an example of a configuration of the lacrimal fluid layer evaluation device according to at least one of the embodiments of the present invention.
Fig. 4 is a flowchart illustrating an example of a flow of determination processing according to at least one of the embodiments of the present invention.
Fig. 5 is an explanatory diagram illustrating an example of a determination target frame extracted from captured video data.
Fig. 6 is an explanatory diagram illustrating an example of setting of a section for extracting sector image data from the extracted determination target frame.
Fig. 7 is an explanatory diagram illustrating an example of displaying a determination result in sector units to be overlapped on the extracted determination target frame.
Fig. 8 is a table in which determination results in frame units are arranged in time series for a plurality of determination target frames.

### Description of Embodiments

Hereinafter, exemplary embodiments of the present invention are described with reference to the drawings. Note that various components in the examples of the respective embodiments described below can be appropriately combined as long as no contradiction or the like occurs. Contents described as an example of a certain embodiment may not be described in other embodiments. Contents of operations and processing not related to characteristics of each embodiment may be omitted. The order of various types of processing configuring various flows described below is not necessarily the same order as long as no contradiction or the like occurs in processing contents.

### [First Embodiment]

Hereinafter, an example of a lacrimal fluid layer evaluation device according to a first embodiment of the present invention is described with reference to the drawings. Note that the lacrimal fluid layer evaluation device of the present invention can be realized as a single lacrimal fluid layer evaluation device that does not need to be connected to other devices via a communication network, but the lacrimal fluid layer evaluation device may be configured to be connected to other devices, for example, a server device or the like via a communication network and cause the other devices to execute a part of processing.

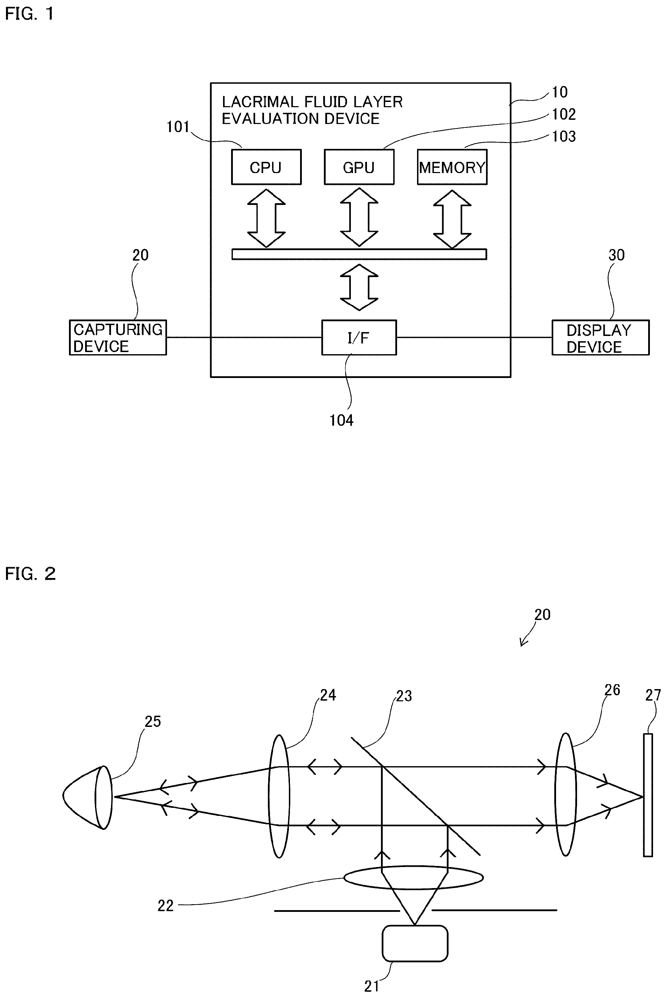

Fig. 1 is a block diagram illustrating an example of a configuration of the lacrimal fluid layer evaluation device and peripheral devices thereof according to at least one of embodiments of the present invention.

A lacrimal fluid layer evaluation device 10 is assumed to be capable of realizing by a general computer, or may be a device designed as a dedicated machine. That is, as illustrated in Fig. 1, the lacrimal fluid layer evaluation device 10 includes a central processing unit (CPU) 101, a graphics processing unit (GPU) 102, and a memory 103 which are usually provided in a general computer. The lacrimal fluid layer evaluation device 10 is connected to a capturing device 20 and a display device 30 via an interface (I/F) 104. An input device such as a mouse or a keyboard, an output device such as a printer, and a communication device for connecting to a communication network may be connected via a bus. Processing in each unit of the lacrimal fluid layer evaluation device 10 is realized by reading a program for executing the processing in each unit from a memory and executing the program in a CPU or a GPU that functions as a control circuit (processing circuit, processing circuitry). In other words, a processor (processing circuit) is configured to be able to execute each processing of each device by executing the program. The capturing device 20 is used to capture an image of an eye to be examined, and the display device 30 is used, for example, to display an image indicating a prediction result to be overlapped on the eye to be examined.

Fig. 2 is an explanatory diagram illustrating an example of a configuration of the capturing device according to at least one of embodiments of the present invention. The capturing device 20 may be any device as long as a video showing the eye to be examined as a capturing target can be stored as digital data, a device known in the related art may be appropriately used, and as schematically illustrated in Fig. 2, in the capturing device 20, light beams emitted from a light source 21 configured with a white LED or the like and passed through a diaphragm are condensed on an anterior eye portion 25 of the eye to be examined of a patient via a lens 22, a splitter 23, and an objective lens 24 in this order. Light reflected from the anterior eye portion 25 passes through the objective lens 24 and the splitter 23 and is imaged on an image capturing element 27 via an imaging lens 26. Captured data imaged on the image capturing element 27 is subjected to predetermined processing by an image processing engine and is converted into still image data and video data. The captured video data captured by the capturing device 20 is transmitted to the lacrimal fluid layer evaluation device 10. Note that, in the following, data may be simply expressed as "image" such as an ophthalmic image and sector image data, and the term is used as an expression including not only contents expressed by "image" but also data of the image. In the following description, it is assumed that the capturing device 20 acquires the captured video data, but data in which time intervals between frames are too long to be regarded as a video and continuity of movement is intermittent, that is, a plurality of pieces of still image data arranged in time series are acquired. However, when the time intervals between frames are long, it is highly likely that eye blinking cannot be detected, and thus it is desirable to reduce the time intervals to several tens of milliseconds or less.

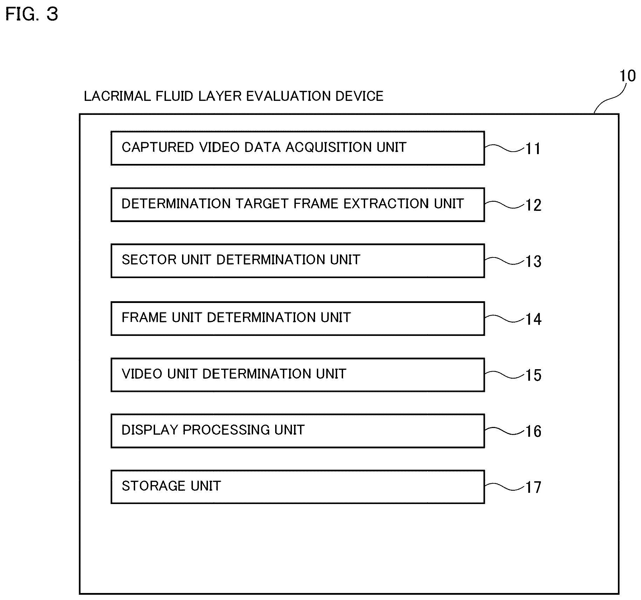

Fig. 3 is a block diagram illustrating an example of a configuration of the lacrimal fluid layer evaluation device according to at least one of embodiments of the present invention. As illustrated in Fig. 3, the lacrimal fluid layer evaluation device 10 includes a captured video data acquisition unit 11, a determination target frame extraction unit 12, a sector unit determination unit 13, a frame unit determination unit 14, a video unit determination unit 15, a display processing unit 16, and a storage unit 17.

The captured video data acquisition unit 11 has a function of acquiring captured video data obtained by capturing the image of the eye to be examined. Any image may be used as long as the image is obtained by capturing the image of the eye to be examined, and for example, it is preferable to include at least one of an opened eyelid section from one closed eyelid to the next closed eyelid. A configuration in which the captured video is divided for each opened eyelid section to obtain each piece of captured video data may be employed.

The determination target frame extraction unit 12 has a function of extracting a plurality of determination target frames from the captured video data based on a predetermined extraction rule. Here, the predetermined extraction rule refers to a rule for extracting a frame as a determination target from a plurality of pieces of frame data configuring captured video data. Generally, in video capturing, the number of frames to be captured per second is set such as 30 fps and 60 fps. It is also possible to set all the frames as the determination target frames, but then, the processing load on the computer increases while state change of the eye to be examined per frame is small, whereby an effect compensating for the increase in processing load may not be obtained. Therefore, adoption of an extraction rule such as extracting one frame at regular time intervals or extracting one frame for every fixed number of frames can be considered. For example, when one determination target frame is extracted every 0.5 seconds in captured video data captured at 30 fps, two determination target frames are obtained every one second.

The sector unit determination unit 13 has a function of extracting a plurality of pieces of sector image data having a predetermined size from the determination target frame and determining whether each piece of sector image data is an image indicating the predetermined lacrimal fluid state. Here, the sector image data refers to image data extracted by cutting out a part having a predetermined size from the determination target frame, the predetermined size being smaller than the size of the determination target frame (size is determined by the number of vertical and horizontal dots). The determination target frame is obtained by capturing an image of the entire eye to be examined, whereas the sector image data is an image showing a small part of the eye to be examined. The predetermined size for extracting the sector image data can be variously set and is preferably set as a size with which whether the lacrimal fluid state is the predetermined lacrimal fluid state is easily determined (with high determination accuracy), because when the predetermined size is too large, the predetermined size includes more pieces of information on portions other than the portion indicating the characteristic of the predetermined lacrimal fluid state whereby it is difficult to specify the portion indicating the characteristic that supports determination, and when the predetermined size is too small, the predetermined lacrimal fluid state characteristic may not be recognized, which leads to erroneous detection. It can be said that the predetermined size is set to different sizes depending on the type of the predetermined lacrimal fluid state. The predetermined lacrimal fluid state refers to a certain state that can be used for evaluation of the eye to be examined. Various lacrimal fluid states may be set as targets, and examples thereof include occurrence of interference fringes in the lacrimal fluid layer, and whether breaks are in the lacrimal fluid layer. It is preferable to be able to determine an area break, a line break, a dimple break, a spot break, or the like that are types of breakup as different lacrimal fluid states.

The sector unit determination unit 13 determines whether each of the plurality of pieces of extracted sector image data corresponds to an image indicating the predetermined lacrimal fluid state. Any method may be used for determining whether the image corresponds to an image indicating the predetermined lacrimal fluid state. As an example, determination based on a learned model that has been learned in advance can be considered. The learning processing of the learned model is performed by extracting sector image data from image data (frame data) for learning obtained by capturing an image of the eye to be examined and predicting the lacrimal fluid state of the eye to be examined for each piece of sector image data by machine learning using correct answer data related to the state of each piece of sector image data. For example, adoption of a convolutional neural network (CNN) as the model to be used can be considered. The sector image data used for learning needs to have the same size as the size of the sector image data extracted by the sector unit determination unit 13. The sector unit determination unit 13 may be specialized for determining one lacrimal fluid state or may be configured so that a plurality of types of lacrimal fluid states can be distinguished and discriminated by one determination processing, and performs learning for the model according to the contents of the determination processing. A configuration in which a plurality of types of lacrimal fluid states are determined by using a plurality of learned models subjected to different learning in combination may be used. As described above, in the learning in which the lacrimal fluid state is determined in sector units, it is possible to improve determination accuracy with a smaller amount of image data than the amount of image data required for learning in the configuration in which the lacrimal fluid state is determined for one entire ophthalmic image.

Note that the learned model used for the determination in the sector unit determination unit 13 may be distributed and provided in a server device as another device, a plurality of terminal devices, or the like so that the lacrimal fluid layer evaluation device is connected to the device provided with the learned model to be used via a communication network each time the learned model is used, other than storing the learned model in the storage unit 17 of the lacrimal fluid layer evaluation device 10. That is, as long as a learned model stored by any storage means can be used, it does not matter whether the storage means of the learned model is provided in the lacrimal fluid layer evaluation device itself or in another device.

When the sector image data is extracted by the sector unit determination unit 13, it is preferable to extract the sector image data continuously for the determination target frame so that a portion indicating the characteristic of the lacrimal fluid state is not erroneously determined. For each piece of the sector image data, the sector image data may be extracted so that at least a part of the image region overlaps with another piece of sector image data. As described above, by extracting the sector image data while overlapping a part thereof, concern of erroneously determining the portion indicating the characteristic of the predetermined lacrimal fluid state existing at a boundary portion between two pieces of sector image data can be reduced.

The frame unit determination unit 14 has a function of determining whether the determination target frame is a candidate image indicating the predetermined lacrimal fluid state based on a predetermined determination condition. As the predetermined determination condition, for example, when the number of pieces of sector image data determined to be an image indicating the predetermined lacrimal fluid state exceeds a predetermined threshold, the determination target frame is determined to be a candidate image indicating the predetermined lacrimal fluid state. Note that the threshold value related to the number of pieces determined to be an image indicating the predetermined lacrimal fluid state in sector units can be set to be different for each type of lacrimal fluid state to be determined. For example, setting a threshold value so that the determination target frame is determined as a candidate image for line break when five pieces in sector units are determined as line break and the determination target frame is determined as a candidate image for spot break when one piece (or two pieces) in sector units is determined as spot break can be considered. Since the set threshold value varies depending on the extraction size of the sector image data, it is preferable to appropriately set an optimum threshold value. The frame unit determination unit 14 performs comparison with a threshold value set for each type of lacrimal fluid state to be determined and determines that the determination target frame is a candidate image indicating the lacrimal fluid state corresponding to the threshold value when the threshold value is exceeded. When determination is performed simultaneously for a plurality of types of lacrimal fluid states, one determination target frame may be determined as a candidate image indicating a plurality of lacrimal fluid states.

The video unit determination unit 15 has a function of determining that the eye to be examined shown in the captured video data is in the predetermined lacrimal fluid state when the determination target frames are arranged in time series and the determination target frames determined to be a candidate image indicating the predetermined lacrimal fluid state satisfy a predetermined continuous condition. Here, the predetermined continuous condition refers to a condition for determining a temporal change for determining as the determination target lacrimal fluid state. As an example, when the determination target frames are arranged in time series, the video unit determination unit 15 determines, as the continuous condition, a case where the determination target frames determined as a candidate image indicating the predetermined lacrimal fluid state continuously appear for a predetermined number or more set in advance. A threshold value regarding how many determination target frames determined as a candidate image are continuous needs to be set to be different for each type of lacrimal fluid state and also needs to be set to be different depending on time intervals of extraction in the determination target frame extraction unit 12. As a specific example, the type of the determination target lacrimal fluid state is assumed to be line break and a criterion for determining that break occurred in the captured video data is that break is continued for 5 seconds, and when extracting two frames per second is set as a predetermined rule and 10 or more determination target frames are continuously determined as a candidate image for line break, it is determined that break occurred in the captured video data.

When the determination target frames are arranged in time series and extracted by the number of frames set in advance at a freely selected point, the video unit determination unit 15 determines, as the continuous condition, a case where the determination target frames determined as a candidate image indicating the predetermined lacrimal fluid state appear by a predetermined ratio or more in an extracted range. For example, when the continuous condition is that a predetermined number or more of frames are continuous but a frame indicating the determination target lacrimal fluid state was not determined for some reason, it is likely that the eye to be examined shown in the captured video data fails to be determined to be in the lacrimal fluid state. Specifically, when the continuous condition is that 10 or more frames are continuous, determination cannot be made by one erroneous determination after seven continuous frames, and then seven continuous frames are determined again, although the frames are actually 15 continuous frames indicating the predetermined lacrimal fluid state, the continuous condition of 10 or more frames being continuous is not satisfied due to one erroneous determination, and consequently, the determination result for the captured video data changes. Here, by determining the continuous condition as that the frames appear in a predetermined ratio or more in a certain range even when the frames are not completely continuous, it is possible to exclude influence of erroneous determination such as noise. As a specific example, setting the continuous condition as that random 10 frames are checked and nine or more frames (90% or more) are determined as a candidate image indicating the predetermined lacrimal fluid state can be considered. Such a continuous condition is effective when the type of lacrimal fluid state is a type in which determination is difficult and erroneous determination occurs at a certain rate.

When the determination target frames are arranged in time series, the video unit determination unit 15 determines, as the continuous condition, a case where the determination target frames determined as a candidate image continuously appear in a period from when the determination target frame determined as a candidate image indicating the predetermined lacrimal fluid state appears until an eyelid is closed. Depending on the type of the lacrimal fluid state, there are states having a characteristic that once the lacrimal fluid state occurs, the lacrimal fluid state continues until the eyelid is closed, and thus the continuous condition corresponds thereto. Breaks as signs of dry eye often have such a characteristic. The continuous condition that the state continues until the eyelid is closed may be applied together with the continuous condition that a predetermined number or more of frames are continuously determined.

The display processing unit 16 has a function of generating a display screen to be displayed on the display device. The display device refers to a display in the lacrimal fluid layer evaluation device or a display connected to the lacrimal fluid layer evaluation device. Generating a display screen for indicating a determination result (evaluation result) can be considered.

The display processing unit 16 may generate a display screen including an image in which a display indicating that the determination target frame is an image indicating the predetermined lacrimal fluid state is displayed to be overlapped on the determination target frame so that a corresponding region of the determination target frame as an extraction source of the sector image data determined as an image indicating the predetermined lacrimal fluid state by the sector unit determination unit 13 can be recognized. According to such processing, a sector portion determined to be in the predetermined lacrimal fluid state such as break can be visually recognized at a glance. As described above, the image is acquired by displaying the display indicating that the determination target frame is an image indicating the predetermined lacrimal fluid state to be overlapped on the determination target frame and a plurality of frames of such images are continuously displayed in time series, whereby temporal change of the predetermined lacrimal fluid state can be visually recognized. When a plurality of types of lacrimal fluid states are simultaneously determined, since different displays are used for different types of lacrimal fluid states, even when displays are simultaneously displayed to be overlapped on the same determination target frame, the types of lacrimal fluid states can be visually distinguished and recognized. Note that, making types of hatching, shades of color, the hue, and the like of different displays to be different from each other can be considered.

When the determination target frames are arranged in time series, the display processing unit 16 may display a graph of temporal change in the number of pieces of sector image data determined to be an image indicating the predetermined lacrimal fluid state.

When the determination target frames are divided into a plurality of aggregation regions and then arranged in time series, the display processing unit 16 may aggregate the number of pieces of sector image data determined as an image indicating the predetermined lacrimal fluid state for each of the aggregation regions and may display a graph of temporal change for each of the aggregation regions. For example, after the eye to be examined is classified into five aggregation regions of a center, an upper side, a lower side, a right side, and a left side, the aggregation region in which the characteristic of the predetermined lacrimal fluid state is increasing is obtained as data, and a graph of the data is displayed as temporal change, whereby a tendency of change in each portion can be read.

The storage unit 17 has a function of storing information necessary for processing of each unit in the lacrimal fluid layer evaluation device 10 and storing various types of information generated by the processing of each unit. When a learned model is used in the sector unit determination unit 13, the learned model may be stored in the storage unit 17. Note that a configuration in which the learned model is stored in a server device connectable via a communication network and the server device includes a function of the sector unit determination unit 13 may be used.

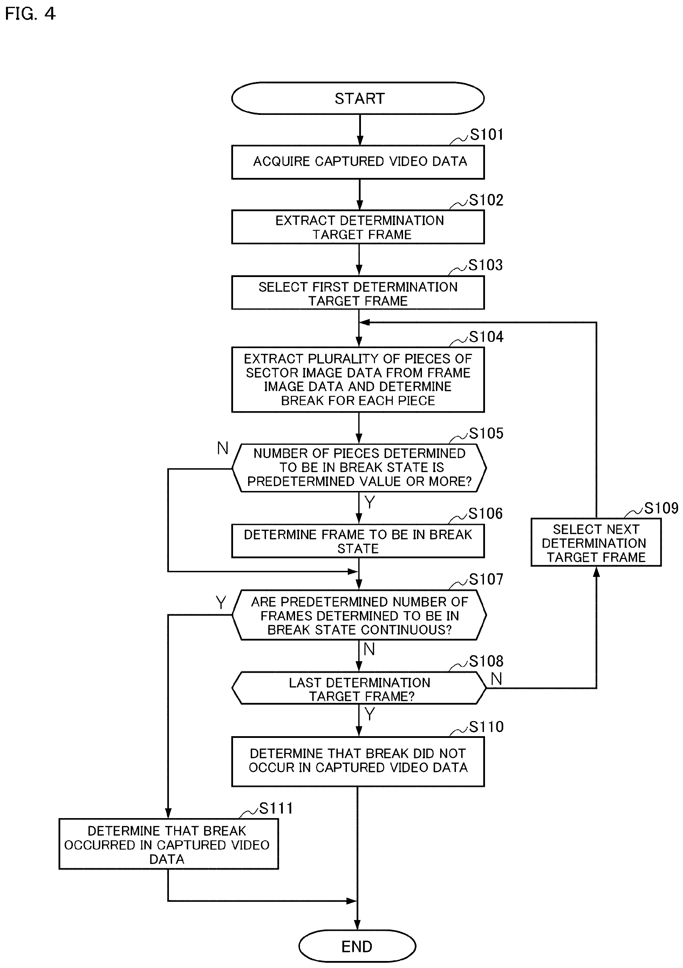

Next, a flow of determination processing according to at least one of embodiments of the present invention is described. Fig. 4 is a flowchart illustrating of an example of the determination processing according to at least one of embodiments of the present invention. In the flowchart illustrated in Fig. 4, an example of determining occurrence of break as a lacrimal fluid state is described. As illustrated in Fig. 4, the determination processing is started by acquiring the captured video data obtained by capturing the image of the eye to be examined by the captured video data acquisition unit 11 of the lacrimal fluid layer evaluation device 10 (step S101). Next, the lacrimal fluid layer evaluation device 10 extracts the plurality of determination target frames from the acquired captured video data by the determination target frame extraction unit 12 (step S102). Next, the lacrimal fluid layer evaluation device 10 selects a first determination target frame by the determination target frame extraction unit 12 (step S103). Next, the lacrimal fluid layer evaluation device 10 extracts the plurality of pieces of sector image data from the image data of the determination target frame and determines whether each piece of sector image data is an image indicating the characteristic of break by the sector unit determination unit 13 (step S104). Next, the lacrimal fluid layer evaluation device 10 determines whether the number of pieces of sector image data determined to be in a break state by the frame unit determination unit 14 is a predetermined threshold value or more (step S105). When the number of pieces of sector image data determined to be in a break state is less than the predetermined threshold value (S105 - N), the process proceeds to step S107. When the number of pieces of sector image data determined to be in a break state is the predetermined threshold value or more (S105 - Y), the lacrimal fluid layer evaluation device 10 determines the determination target frame as a candidate image for break by the frame unit determination unit 14 (step S106). Next, the lacrimal fluid layer evaluation device 10 determines whether a predetermined number of determination target frames determined to be in a break state are continuous by the video unit determination unit 15 (step S107). When the predetermined number of determination target frames determined to be in a break state are not continuous (S107 - N), it is determined whether the determination target frame is the last determination target frame (step S108). When the determination target frame is not the last determination target frame (S108 - N), the next determination target frame is selected (step S109), and then the process proceeds to step S104. As such, the processes of steps S104 to S109 are repeatedly executed until the predetermined number of determination target frames determined to be in a break state are continuous (S107 - Y) or the determination target frame is the last determination target frame (S108 - Y).

During repetition of the processing, when the predetermined number of determination target frames determined to be in a break state are continuous (S107 - Y), the lacrimal fluid layer evaluation device 10 determines that break occurred in the captured video data by the video unit determination unit 15 (step S111) and ends the determination processing. During repetition of the processing, when the predetermined number of determination target frames determined to be in a break state are not continuous (S107 - N) and the processing is completed for the last determination target frame (S108 - Y), it is determined that no break occurred in the captured video data (step S110), and the determination processing ends.

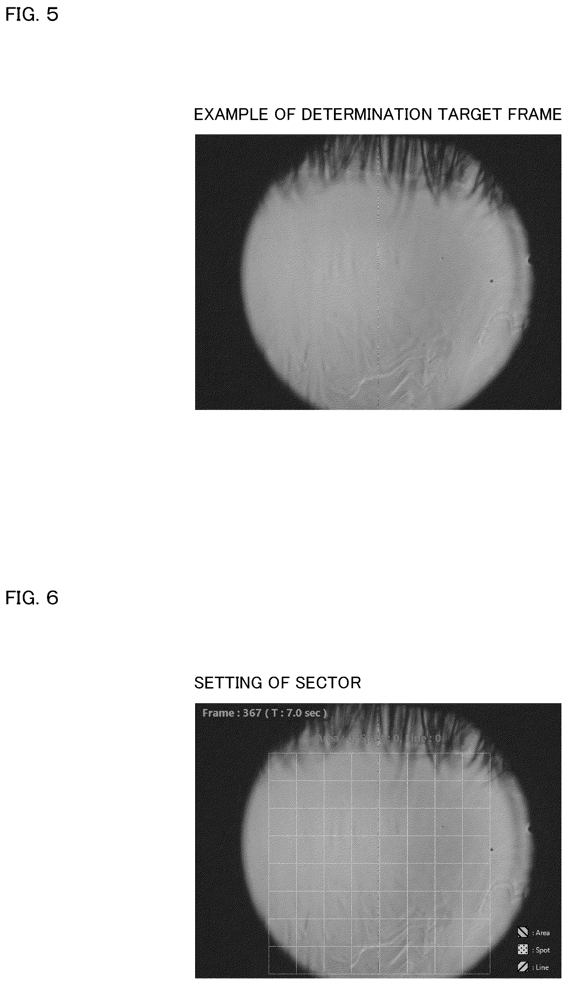

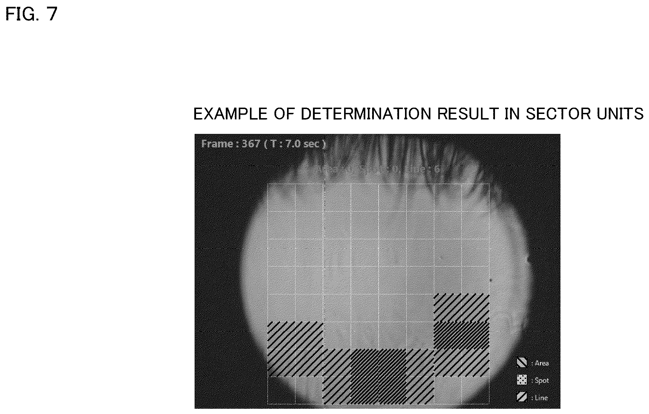

Next, the determination of the lacrimal fluid state in sector units is described using the drawings. Fig. 5 is an explanatory diagram illustrating an example of the determination target frame extracted from captured video data. Fig. 6 is an explanatory diagram illustrating an example of setting of a section for extracting sector image data from the extracted determination target frame. In the example illustrated in Fig. 7, a total of 49 sections of seven in length × seven in width are set for the eye to be examined, and a total of four sections of two in length × two in width are extracted as one piece of sector image data. Here, the sector image data is extracted while overlapping with another piece of adjacent sector image data in two sections. Then, it is determined for each piece of sector image data whether the image data is an image indicating a lacrimal fluid state. Fig. 7 is an explanatory diagram illustrating an example of displaying a determination result in sector units to be overlapped on the extracted determination target frame. In the example of Fig. 7, it is shown that six pieces of sector image data are determined to be in a line break state as a result of performing determination for area break, spot break, and line break, and in portions where the determination result is displayed to be overlapped, portions overlapping in the adjacent sector image data are drawn to have a narrower interval of oblique lines. In Fig. 7, the portions where the determination results overlap with each other are visually distinguished by different intervals between oblique lines, but the portions where the determination results overlap with each other may be distinguished by different shades of color.

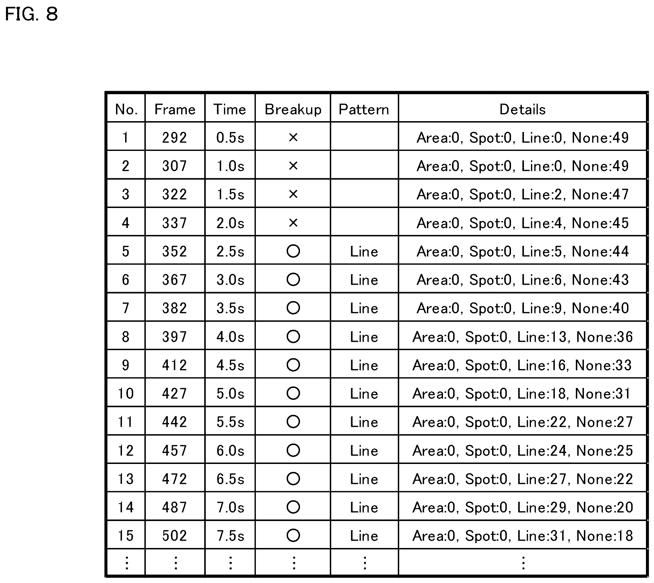

Next, the determination of the lacrimal fluid state in video units is described. Fig. 8 is a table in which determination results in frame units are arranged in time series for a plurality of determination target frames. The first column shows the numbering of the determination target frame, the second column shows the frame number, the third column shows the time in the captured video data (seconds), the fourth column shows the determination result of break (lacrimal fluid state), the fifth column shows the content of the determined pattern, and the sixth column shows the details of the determination result in sector units. In the example of Fig. 8, when the number of images determined to be in a line break state in sector units is five or more, the determination target frame is determined as a candidate image for line break. In the example of Fig. 8, it is determined that there is no break up to the fourth determination target frame, but since the number of images determined to be in a line break state in sector units in the fifth determination target frame is five, the fifth determination target frame is determined as a candidate image for break. Thereafter, the number of images determined to be in a line break state in sector units tends to gradually increase from the sixth to the fifteenth, and since there are five or more breaks in sector units, the images are continuously determined to be a candidate image for break even in frame units. In such a situation, for example, when 10 determination target frames are continuously determined to be a candidate image for break and the continuous condition for determining that break occurred in the captured video data is adopted, since the condition of 10 consecutive times is satisfied in the determination target frame No. 14, by determining the determination target frame No. 14 as a candidate image for break, the shadow video data is determined that break occurred therein.

As described above, a first aspect of the first embodiment includes a captured video data acquisition unit that acquires captured video data obtained by capturing an image of an eye to be examined, a determination target frame extraction unit that extracts a plurality of determination target frames from the captured video data based on a predetermined extraction rule, a sector unit determination unit that extracts a plurality of pieces of sector image data having a predetermined size from the determination target frame and determines whether each piece of sector image data is an image indicating a predetermined lacrimal fluid state, a frame unit determination unit that determines that the determination target frame is a candidate image indicating the predetermined lacrimal fluid state when the number of pieces of sector image data determined as an image indicating the predetermined lacrimal fluid state exceeds a predetermined threshold value, and a video unit determination unit that determines that the eye to be examined shown in the captured video data is in the predetermined lacrimal fluid state when the determination target frames are arranged in time series and the determination target frames determined as a candidate image indicating the predetermined lacrimal fluid state satisfy a predetermined continuous condition, and thus determination accuracy is improved by subdividing a captured image and the lacrimal fluid layer can be evaluated considering temporal changes of the lacrimal fluid layer.

That is, even when an image in frame units is determined to be in the predetermined lacrimal fluid state, since determination can be erroneous or determination may not be accurate when using only one frame as the determination result, there is a problem that determination that the eye to be examined shown in the captured video data is in the predetermined lacrimal fluid state cannot be immediately made, but as the present example has a configuration in which the eye to be examined shown in the captured video data is determined to be in the predetermined lacrimal fluid state when the continuous condition for determining the predetermined lacrimal fluid state is satisfied, it is possible to improve determination accuracy. Determination on each determination target frame in frame units is also made using the determination result in sector units, so that accuracy of the determination result in frame units can be improved.

### [Second Embodiment]

In the first embodiment, as a premise of determination in the frame unit determination unit 14, the sector unit determination unit 13 performs determination in sector units, but the present invention is not necessarily limited thereto.

That is, without performing determination in sector units, determination whether a frame is a candidate image indicating the predetermined lacrimal fluid state may be directly performed on the entire determination target frame. Using a learned model for such a determination can be considered, but if learning accuracy in frame units is improved along with technical improvement in the technical fields of machine learning and deep learning, even in a configuration in which determination of whether the frame is a candidate image indicating the predetermined lacrimal fluid state is directly performed for the entire determination target frame, it is likely that determination accuracy for the captured video data similar to that of the first embodiment can be realized.

### Reference Signs List

- 10: lacrimal fluid layer evaluation device
- 11: captured video data acquisition unit
- 12: determination target frame extraction unit
- 13: sector unit determination unit
- 14: frame unit determination unit
- 15: video unit determination unit
- 16: display processing unit
- 17: storage unit
- 20: capturing device
- 21: light source
- 22: lens
- 23: splitter
- 24: objective lens
- 25: anterior eye portion
- 26: imaging lens
- 27: image capturing element
- 30: display device
- 101: CPU
- 102: GPU
- 103: memory
- 104: I/F

## Claims

1. A lacrimal fluid layer evaluation device for evaluating a lacrimal fluid state of an eye to be examined based on captured video data obtained by capturing an image of the eye to be examined, the device comprising:
a captured video data acquisition unit that acquires the captured video data obtained by capturing the image of the eye to be examined;
a determination target frame extraction unit that extracts a plurality of determination target frames from the captured video data based on a predetermined extraction rule;
a frame unit determination unit that determines whether the determination target frame is a candidate image indicating a predetermined lacrimal fluid state based on a predetermined determination condition; and
a video unit determination unit that determines that the eye to be examined shown in the captured video data is in the predetermined lacrimal fluid state when the determination target frames are arranged in time series and the determination target frames determined as a candidate image indicating the predetermined lacrimal fluid state satisfy a predetermined continuous condition.

2. The lacrimal fluid layer evaluation device according to claim 1, further comprising
a sector unit determination unit that extracts a plurality of pieces of sector image data having a predetermined size from the determination target frame and determines whether each piece of sector image data is an image indicating the predetermined lacrimal fluid state, wherein
when the number of pieces of sector image data determined as an image indicating the predetermined lacrimal fluid state exceeds a predetermined threshold value, the frame unit determination unit determines that the determination target frame is a candidate image indicating the predetermined lacrimal fluid state.

3. The lacrimal fluid layer evaluation device according to claim 2,
wherein the frame unit determination unit sets and stores a threshold value relating to the number of pieces of sector image data for determining that the determination target frame is an image indicating the predetermined lacrimal fluid state for each type of a lacrimal fluid state as a determination target.

4. The lacrimal fluid layer evaluation device according to claim 2,
wherein the sector unit determination unit extracts the sector image data so that at least a part of an image region is overlapped on another piece of sector image data for each of the plurality of pieces of sector image data.

5. The lacrimal fluid layer evaluation device according to claim 2, further comprising
a display processing unit that generates a display screen to be displayed on a display device, wherein
the display processing unit displays a display indicating that the determination target frame is an image indicating the predetermined lacrimal fluid state to be overlapped on the determination target frame so that a corresponding region of the determination target frame as an extraction source of the sector image data determined as an image indicating the predetermined lacrimal fluid state by the sector unit determination unit can be recognized.

6. The lacrimal fluid layer evaluation device according to claim 2, further comprising
a display processing unit that generates a display screen to be displayed on a display device, wherein
when the determination target frames are arranged in time series, the display processing unit displays a graph of temporal change in the number of pieces of sector image data determined as the image indicating the predetermined lacrimal fluid state.

7. The lacrimal fluid layer evaluation device according to claim 6,
wherein, when the determination target frames are divided into a plurality of aggregation regions and then arranged in time series, the display processing unit aggregates the number of pieces of sector image data determined as an image indicating the predetermined lacrimal fluid state for each of the aggregation regions and displays a graph of temporal change for each of the aggregation regions.

8. The lacrimal fluid layer evaluation device according to any one of claims 1 to 7,
wherein, when the determination target frames are arranged in time series, a video unit determination unit determines, as the continuous condition, a case where the determination target frames determined as a candidate image indicating the predetermined lacrimal fluid state continuously appear for a predetermined number or more set in advance.

9. The lacrimal fluid layer evaluation device according to any one of claims 1 to 7,
wherein, when the determination target frames are arranged in time series and are extracted by the number of frames set in advance at a freely selected point, a video unit determination unit determines, as the continuous condition, a case where the determination target frames determined as a candidate image indicating the predetermined lacrimal fluid state appear by a predetermined ratio or more in an extracted range.

10. The lacrimal fluid layer evaluation device according to any one of claims 1 to 7,
wherein, when the determination target frames are arranged in time series, a video unit determination unit determines, as the continuous condition, a case where the determination target frames determined as a candidate image continuously appear in a period from when the determination target frame determined as a candidate image indicating the predetermined lacrimal fluid state appears until an eyelid is closed.

11. A lacrimal fluid layer evaluation program for causing a computer to realize processing of evaluating a lacrimal fluid state of an eye to be examined based on captured video data obtained by capturing an image of the eye to be examined, the program causing the computer to realize:
a captured video data acquisition function of acquiring the captured video data obtained by capturing the image of the eye to be examined;
a determination target frame extraction function of extracting a plurality of determination target frames from the captured video data based on a predetermined extraction rule;
a frame unit determination function of determining whether the determination target frame is a candidate image indicating a predetermined lacrimal fluid state based on a predetermined determination condition; and
a video unit determination function of determining that the eye to be examined shown in the captured video data is in the predetermined lacrimal fluid state when the determination target frames are arranged in time series and the determination target frames determined as a candidate image indicating the predetermined lacrimal fluid state satisfy a predetermined continuous condition.

12. A lacrimal fluid layer evaluation method for evaluating a lacrimal fluid state of an eye to be examined based on captured video data obtained by capturing an image of the eye to be examined by a computer, the method comprising:
a captured video data acquisition step of acquiring the captured video data obtained by capturing the image of the eye to be examined;
a determination target frame extraction step of extracting a plurality of determination target frames from the captured video data based on a predetermined extraction rule;
a frame unit determination step of determining whether the determination target frame is a candidate image indicating a predetermined lacrimal fluid state based on a predetermined determination condition; and
a video unit determination step of determining that the eye to be examined shown in the captured video data is in the predetermined lacrimal fluid state when the determination target frames are arranged in time series and the determination target frames determined as a candidate image indicating the predetermined lacrimal fluid state satisfy a predetermined continuous condition.
